# EUROPEAN PATENT APPLICATION

(11) **EP 3 788 965 A1**
(43) Date of publication of application: **10.03.2021**
(21) Application number: 20194021.0
(22) Date of filing: 02.09.2020
(51) Int. Cl.: A61B 17/072, A61B 17/00

(54) **LOADING AND RESETTING STRUCTURE OF THE ENDOSCOPIC CUTTING STAPLER**

(30) Priority: 05.09.2019 CN 201910836828
(71) Applicant: Jiangsu Guanchuang Medical Technology Co., Ltd., Changzhou City, Jiangsu Province (CN)
(72) Inventor: Lv, Bo, Changzhou City, Jiangsu Province (CN)
(74) Representative: Cabinet Chaillot

(57) **Abstract**

The invention relates to a loading and resetting structure of the endoscopic cutting stapler assembly wherein endoscopic cutting stapler assembly comprises connecting rod assembly, staple cartridge assembly, and tiltable and repositionable anvil assembly; the connecting rod assembly is cylindrical in shape comprising lower fixing element and upper fixing element detachably connected with the other end of lower fixing element; the connector for connecting the endoscope body is provided at one end of connecting rod assembly away from staple cartridge assembly, and an arc-shaped groove is arranged along peripheral faces of the connecting rod assembly closing to connector, the height of which less than or equal to 1.1mm; the loading and resetting structure includes rotating element and elastic reset element wherein rotating element is arranged on the arc-shaped groove, and one end of elastic reset element is fixed on rotating element while the other end thereof is fixed on arc-shaped groove.

## Description

### BACKGROUND OF THE INVENTION

### 1. Field of the Invention

The invention relates to the technical field of the endoscopic cutting stapler, specifically relating to a loading and resetting structure of the endoscopic cutting stapler assembly.

### 2. Description of the Related Art

The endoscopic cutting stapler and disposable staple cartridge are applicable to the ablation, transection and anastomosis of tissues in abdominal, gynecologic, paediatric and thoracic surgery, which can also be applied in the ablation and transection of liver parenchyma, liver vessels, and cystic duct. The endoscopic cutting stapler consists of the endoscopic cutting stapler assembly and the endoscope body wherein one end of the endoscopic cutting stapler is provided with a connector for connecting the endoscope body and a rotating element is arranged closing to the connector; the rotating element has two functions: firstly, rotating to the assembly position to facilitate connection with the endoscope body and prevent the triggered endoscopic cutting stapler assembly from failure to unload from the endoscope body caused by abnormal loading of the endoscopic cutting stapler assembly(the cutting knife assembly has been ejected); secondly, when the rotating element is not at initial assembly position, the endoscopic cutting stapler assembly still in an incompletely prepared state and cannot be loaded.

At present, the rotating element can be rotated freely, therefor it needs to be rotated to the original assembly position manually, which is troublesome and prone to cause mistake or mistaken touch; once misoperation or mistaken touch occur, the misplaced endoscopic cutting stapler assembly cannot reset automatically and when the endoscopic cutting stapler assembly with wrong status is circulated to the hands of its users, it will be unable to normally load and use; however, when rotated to the original assembly position manually, the position of the endoscopic cutting stapler assembly may beyond a bit distance from the prepared state and not in a prepared state, the abnormal loading of the endoscopic cutting stapler assembly causing the triggered endoscopic cutting stapler assembly failure to unload from the endoscope body, bearing great safety hazard.

### SUMMARY OF THE INVENTION

The technical problem that the invention aims to solve is to overcome the shortcomings in the prior art and to provide a loading and resetting structure of the endoscopic cutting stapler assembly.

The invention adopts following technical solution to solve the technical problem: a loading and resetting structure of the endoscopic cutting stapler assembly wherein the endoscopic cutting stapler assembly comprises a connecting rod assembly, a staple cartridge assembly connected on the one end of the connecting rod assembly, and a tiltable and repositionable anvil assembly among which the connecting rod assembly is cylindrical in shape comprising a lower fixing element and an upper fixing element which is detachably connected with the other end of the lower fixing element; the connector for connecting the endoscope body is provided at one end of the connecting rod assembly that is away from the staple cartridge assembly, and an arc-shaped groove is arranged along the peripheral faces of the connecting rod assembly closing to the connector, the height of which less than or equal to 1.1mm; the loading and resetting structure includes a rotating element and an elastic reset element wherein the rotating element is arranged on the arc-shaped groove, and one end of the elastic reset element is fixed on the rotating element while the other end thereof is fixed on the arc-shaped groove.

Further defined, an arc-shaped projection and a first connecting block are arranged in the middle of the arc-shaped groove, said first connecting block is located at one end of the arc-shaped projection.

Further defined, an arc-shaped hole for accommodating the elastic reset element is formed in the middle of the arc-shaped projection.

Further defined, the rotating element is provided with a groove matched with the arc-shaped projection in the middle, and the rotating element at one end of the groove is provided with a second connecting block.

It is still further defined that one end of the elastic reset element is connected to the first connecting block, and the other end thereof is connected to the second connecting block.

Further defined, the height of the elastic reset element is less than or equal to 1.1mm, and the elastic reset element comprises a tension spring, a pressure spring, a torsion spring or a high polymer material elastic piece.

Further defined, the connecting rod assembly is equipped with the sleeve outward, and the connector stretches out of the sleeve.

The invention has following advantageous effects: compared with the prior art, by adding the loading and resetting mechanism, the rotating element can be reset to the assembly position without manual operation and then connected with the endoscope body, reducing the procedures of manual reset and checking whether to reset, saving both time and labour; the elastic reset element can keep the rotating element in a complete prepared state, thus eliminating the potential safety hazard due to failure to load the endoscopic cutting stapler assembly normally caused by misoperation or mistaken touch of the rotating element in the homogeneous endoscopic cutting stapler assembly products without reset mechanism.

### BRIEF DESCRIPTION OF THE DRAWINGS

The invention is further described in detail hereinafter with reference to the drawings and embodiment.
FIG.1 is the assembly diagram of the invention.
FIG.2 is the structural diagram of the invention removing sleeve.
FIG.3 is an enlarged view of the I section in FIG.1.
FIG.4 is the structural diagram of the rotating element in the invention.
FIG.5 is the top view of FIG.4.

In the Figures, 1. connecting rod assembly, 2. staple cartridge assembly, 3. tiltable and repositionable anvil assembly, 4. rotating element, 5. elastic reset element, 6. sleeve, 11. lower fixing element, 12. upper fixing element, 121. connector, 122. arc-shaped groove, 123. arc-shaped projection, 124. first connecting block, 1231. arc-shaped hole, 41. groove, 42. second connecting block.

### DETAILED DESCRIPTION OF THE PREFERRED EMBODIMENTS

The invention is further described in detail hereinafter with reference to the drawings and preferred embodiment. These drawings are simplified diagrams each illustrating the basic structure of the invention only in a schematic manner, and thus showing only the constitution related to the invention.

### Embodiment 1

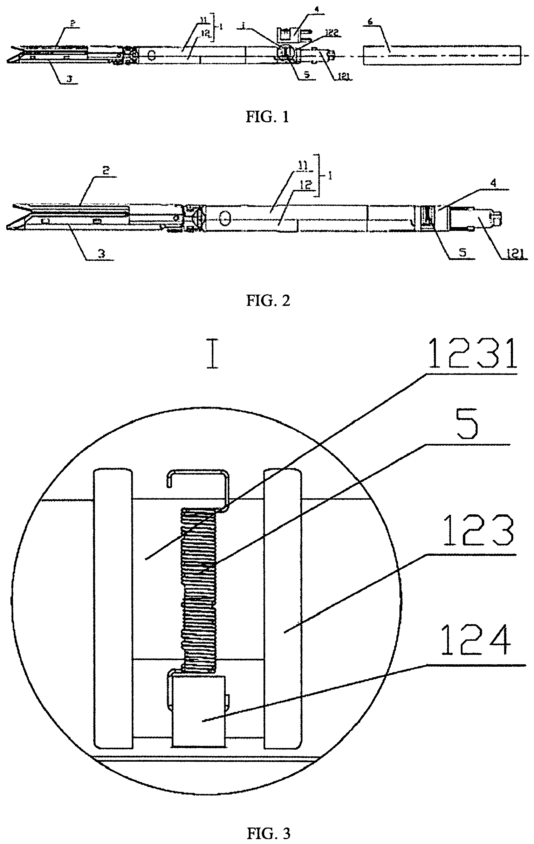

As shown in FIG.1∼2, the embodiment discloses a a loading and resetting structure of the endoscopic cutting stapler assembly wherein the endoscopic cutting stapler assembly comprises a connecting rod assembly 1, a staple cartridge assembly 2 connected on the one end of the connecting rod assembly 1, and a tiltable and repositionable anvil assembly 3 among which the connecting rod assembly 1 is cylindrical in shape comprising a lower fixing element 11 and an upper fixing element 12 which is detachably connected with the other end of the lower fixing element 11; the connector 121 for connecting the endoscope body is provided at one end of the upper fixing element 12 that is away from the staple cartridge assembly 2; the connecting rod assembly 1 is equipped with the sleeve 6 outward, and the connector 121 stretches out of the sleeve 6, and an arc-shaped groove 122 is arranged along the peripheral faces of the upper fixing element 12 closing to the connector 121, the height of which less than or equal to 1.1mm; the loading and resetting structure includes a rotating element 4 and an elastic reset element 5 wherein the rotating element 4 is arranged on the arc-shaped groove 122, and one end of the elastic reset element 5 is fixed on the rotating element 4 while the other end thereof is fixed on the arc-shaped groove 122.

In addition, the arc-shaped groove 122 can also be arranged on the lower fixing element 11; one end of the elastic reset element 5 is fixed on the rotating element 4 while the other end thereof can be fixed on the upper fixing element 12 or the lower fixing element 11 so as to realize automatic reset after rotation of the rotating element 4.

As shown in FIG.3, an arc-shaped projection 123 and a first connecting block 124 are arranged in the middle of the arc-shaped groove 122, said first connecting block 124 is located at one end of the arc-shaped projection 123. An arc-shaped hole 1231 for accommodating the elastic reset element 5 is formed in the middle of the arc-shaped projection 123.

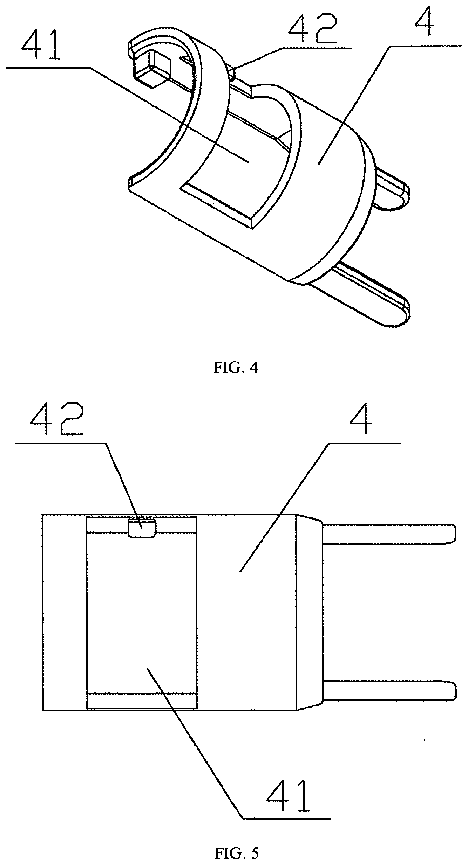

As shown in FIG.4∼5, the rotating element 4 is provided with a groove 41 matched with the arc-shaped projection 123 in the middle, and the rotating element 4 at one end of the groove 41 is provided with a second connecting block 42. One end of the elastic reset element 5 is connected to the first connecting block 124, and the other end thereof is connected to the second connecting block 42.

The height of the elastic reset element 5 is less than or equal to 1.1mm, and the elastic reset element 5 comprises a tension spring, a pressure spring, a torsion spring or a high polymer material elastic piece, but does not limited thereto.

During specific installation, the upper fixing element 12 and the lower fixing element 11 are respectively connected with a connecting sleeve via the steering joint, the tiltable and repositionable anvil assembly 3 is connected with the connecting sleeve fixedly, the staple cartridge assembly 2 is rotatably connected to the tiltable and repositionable anvil assembly 3; the sleeve 6 is fixed on the lower fixing element 11, the rotating element 4 is set on the arc-shaped groove 122 and the groove 41 cooperates with the arc-shaped projection 123; one end of the elastic reset element 5 is connected on the first connecting block 124 in the middle of the arc-shaped groove 122 and the other end thereof is connected to the second connecting block 42 of the rotating element 4; the rotating element 4 after the assembly is concentric with the common axle center of the cylindrical connecting rod assembly 1 and can rotate along this axle center at certain angle.

The invention and its embodiments have been described above, but the description is not limited thereto; only one embodiment of the invention is shown in the drawings, and the actual structure is not limited thereto. In general, it is to be understood by those skilled in the art that non-creative design of structural forms and embodiments that are similar to the technical solutions without departing from the spirit of the invention shall all fall within the protective scope of the invention.

## Claims

1. A loading and resetting structure of the endoscopic cutting stapler assembly wherein the endoscopic cutting stapler assembly comprises a connecting rod assembly, a staple cartridge assembly connected on the one end of the connecting rod assembly, and a tiltable and repositionable anvil assembly among which the connecting rod assembly is cylindrical in shape comprising a lower fixing element and an upper fixing element which is detachably connected with the other end of the lower fixing element; the connector for connecting the endoscope body is provided at one end of the connecting rod assembly that is away from the staple cartridge assembly, and an arc-shaped groove is arranged along the peripheral faces of the connecting rod assembly closing to the connector, the height of which less than or equal to 1.1mm; it is **characterized in that** said loading and resetting structure includes a rotating element and an elastic reset element wherein said rotating element is arranged on the arc-shaped groove, and one end of the elastic reset element is fixed on the rotating element while the other end thereof is fixed on the arc-shaped groove.

2. The loading and resetting structure of the endoscopic cutting stapler assembly of claim 1 wherein an arc-shaped projection and a first connecting block are arranged in the middle of the arc-shaped groove and said first connecting block is located at one end of said arc-shaped projection.

3. The loading and resetting structure of the endoscopic cutting stapler assembly of claim 2 wherein an arc-shaped hole for accommodating the elastic reset element is formed in the middle of said arc-shaped projection.

4. The loading and resetting structure of the endoscopic cutting stapler assembly of claim 2 wherein said rotating element is provided with a groove matched with the arc-shaped projection in the middle, and the rotating element at one end of said groove is provided with a second connecting block.

5. The loading and resetting structure of the endoscopic cutting stapler assembly of claim 4 wherein one end of said elastic reset element is connected to the first connecting block, and the other end thereof is connected to the second connecting block.

6. The loading and resetting structure of the endoscopic cutting stapler assembly of claim 1 or 4 wherein the height of said elastic reset element is less than or equal to 1.1mm, and the elastic reset element comprises a tension spring, a pressure spring, a torsion spring or a high polymer material elastic piece.

7. The loading and resetting structure of the endoscopic cutting stapler assembly of claim 1 wherein said connecting rod assembly is equipped with the sleeve outward, and the connector stretches out of the sleeve.
